(19) Europäisches Patentamt
European Patent Office
Office européen des brevets

(11) **EP 4 236 906 B1**

(12) **EUROPEAN PATENT SPECIFICATION**

(45) Date of publication and mention
of the grant of the patent:
**06.08.2025   Bulletin 2025/32**

(21) Application number: **21811629.1**

(22) Date of filing: **28.10.2021**

(51) International Patent Classification (IPC):
*A61K 8/02* (2006.01)        *A61K 8/25* (2006.01)
*A61Q 17/04* (2006.01)        *C01B 33/18* (2006.01)

(52) Cooperative Patent Classification (CPC):
**A61K 8/0279; A61K 8/25; A61Q 17/04;**
**C01B 33/18;** A61K 2800/412

(86) International application number:
**PCT/US2021/056968**

(87) International publication number:
**WO 2022/094022 (05.05.2022 Gazette 2022/18)**

(54) **ASPHERICAL HOLLOW SILICA PARTICLES AS SPF BOOSTERS**

ASPHÄRISCHE HOHLE KIESELSÄURETEILCHEN ALS SPF-VERSTÄRKER

PARTICULES DE SILICE CREUSES ASPHÉRIQUES UTILISÉES COMME RENFORÇATEURS DE FACTEURS DE PROTECTION SOLAIRE (SPF)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **28.10.2020   US 202063106645 P**

(43) Date of publication of application:
**06.09.2023   Bulletin 2023/36**

(73) Proprietors:
• **Dow Global Technologies, LLC**
**Midland, MI 48674 (US)**
• **Rohm and Haas Company**
**Collegeville, PA 19426 (US)**

(72) Inventors:
• **XIONG, Jie**
**Lake Jackson, TX 77566 (US)**
• **ZENG, Fanwen**
**Collegeville, PA 19426 (US)**
• **XU, Wenjun**
**Collegeville, PA 19426 (US)**
• **MILLAR, Dean, M.**
**Midland, MI  48667 (US)**

(74) Representative: **Beck Greener LLP**
**Fulwood House**
**12 Fulwood Place**
**London WC1V 6HR (GB)**

(56) References cited:
**WO-A1-2014/203913     US-A1- 2020 237 632**

• **MASAYOSHI FUJI ET AL: "Development of new templating approach for hollow nanoparticles and their applications", ADVANCED POWDER TECHNOLOGY, vol. 25, no. 1, 1 January 2014 (2014-01-01), NL, pages 91 - 100, XP055570123, ISSN: 0921-8831, DOI: 10.1016/j.apt.2013.12.002**

**Description**

**CROSS-REFERENCE TO RELATED APPLICATIONS**

This application claims the benefit of U.S. Provisional Patent Application No. 63/106,645, filed October 28, 2020.

**BACKGROUND**

Sun protection factor (SPF) is used to rate a sun care composition's ability to block, absorb, and/or scatter UV radiation (e.g., UVA radiation and/or UVB radiation). A sun care composition may contain physical UV blockers and/or chemical UV absorbers which are described herein as sunscreen actives. However, too high a concentration of sunscreen active results in impairment of the sun care composition's aesthetics and/or engenders undesirable toxicological effects and/or environmental issues. Consequently, SPF boosters (e.g., compounds which are not recognized sunscreen actives, but work to increase the SPF) are highly desirable for addition to sun care compositions, for example, to increase the SPF of a sun care composition without adding more sunscreen actives.

US2020237632 A1 discloses a UV screening composition comprising UV filters and a UV booster (ie. SPF booster) which is a spherical silica having a particle size of 1 to 25 microns. The spherical silica may be coated or uncoated, porous or non-porous, and may be hollow. WO2014203913 A1 discloses a sunscreen composition comprising an SPF booster such as hollow silica particles, and a uv screening agent.

Masayoshi Fuji ET AL: "Development of new templating approach for hollow nanoparticles and their applications", ADVANCED POWDER TECHNOLOGY, vol. 25, no. 1, 01.01.2014, pages 91-100 is a review of templating methods for forming hollow nanoparticles. Section 3 discusses the various approaches using solid core templating, including the use of calcium carbonate templates to form micro or nano sized hollow particles.

Accordingly, what is needed are new SPF boosters and new processes for forming the same.

**SUMMARY**

Described herein are aspherical hollow silica particles, processes for making aspherical hollow silica particles, and uses of aspherical hollow silica particles in sun care compositions. To form the aspherical hollow silica particles, deposition of a silica shell on a calcium carbonate template using a sol-gel chemistry is employed. Subsequent dissolution of the calcium carbonate template forms voids (e.g., a hollow interior) in the aspherical hollow silica particles.

**BRIEF DESCRIPTION OF THE DRAWINGS**

FIG. 1 is a group of Scanning Transmission Electron Microscope (STEM) images of aspherical hollow silica particles labeled Batches 1-5.

FIG. 2 is an Attenuated Total Reflection Fourier Transform Infrared Spectroscopy (ATR-FTIR) spectra of Batch 1 aspherical hollow silica particles at room temperature and after drying, along with a reference spectra of tetraethyl orthosilicate (TEOS).

FIG. 3 is a diagram of sun protection factor (SPF) measurements for initial and heat aged formulations including comparative sun care formulations (e.g., no SPF booster or a conventional SPF booster), a sun care formulation incorporating template material, and a sun care formulation that includes aspherical hollow silica particles (e.g., as an SPF booster).

**DETAILED DESCRIPTION**

Described herein are aspherical hollow silica particles, processes for making aspherical hollow silica particles, and uses of aspherical hollow silica particles in sun care compositions. "Aspherical," with respect to the aspherical hollow silica particles described herein, means that the particles are not generally spherical. Preferably, the aspherical hollow silica particles have an anisotropic shape (e.g., a long axis and a short axis). "Hollow," with respect to the aspherical hollow silica particles described herein, means that the particles have a void (e.g., hollow interior portion) defined by a shell of silicon oxide particles. A plurality of pores (e.g., channels) may pass through the shell, extending from the hollow interior portion to the exterior surface of the shell.

Aspherical hollow silica particles may be prepared by depositing silica (e.g., via a sol-gel process) on an inorganic template. The silica may be from silicate precursors, such as, for example, alkoxy silanes, alkyl silicates, etc. Preferably, the process for making aspherical hollow silica particles comprises obtaining calcium carbonate crystals for use as a template, depositing silica shells on the template, and then dissolving the template with acid, to afford aspherical hollow silica particles. More preferably, tetraethyl orthosilicate is used to form the silica shells. The morphology of the aspherical hollow silica particles may be a result of the morphology of the inorganic template, for example, the silica may be deposited relatively evenly, for example as a continuous silica shell. The inorganic template may be dissolved with acid. The inorganic template may be calcium carbonate. The aspherical hollow silica particles may contain less than about 2 wt.% organic substances, preferably less than about 1.5 wt.% organic substances, and more preferably, preferably less than about 1.0 wt.% organic substances.

The process may be a surfactant-free process.

In another embodiment, it is contemplated to vary the process conditions used to create the inorganic template to afford spherical calcium carbonate crystals for use as a template. The above described process, e.g., depositing silica (e.g., via a sol-gel process) on the inorganic template, would then be followed to afford spherical hollow silica particles.

A preferred process for making aspherical hollow silica particles comprises dispersing calcium carbonate templates in ethanol, adding ammonium solution, then slowly adding tetraethyl orthosilicate (TEOS), and adding acid to dissolve the calcium carbonate templates. The process may include quenching the reaction with ethanol before the step of adding acid. The process may include between about 30 minutes to about 90 minutes before quenching the reaction with ethanol. The aspherical hollow silica particles may contain less than about 2 wt.% organic substances without further purification.

The calcium carbonate template material may be formed by slowly adding a calcium chloride solution to a sodium carbonate solution in the presence of ethylene glycol. After a period of stirring, the product (calcium carbonate) is separated by centrifugation. The centrifuged product is washed with ethanol. The concentration of the reagents, reaction time, and temperature may have an impact on the template size.

Aspherical hollow silica particles described herein preferably have an anisotropic shape (e.g., a long axis and a short axis). Scanning Transmission Electron Microscope (STEM) images may be used to determine particle morphology measuring manually using a scale bar. For example, STEM may be used to observe the length of the long axis and the short axis, presence of a hollow void, dimensions of the void, and shell thickness, measuring manually using a scale bar.

The aspherical hollow silica particles have a long axis (e.g., a maximum outer particle length) of about 450 nm to about 1650 nm. Preferably, the long axis may be greater than about 800 nm, greater than about 1000 nm, greater than about 1100 nm, and less than about 1227 nm, less than about 1300 nm, and less than about 1422 nm. More preferably, the long axis of the aspherical hollow silica particles may be about 1100 nm to about 1200 nm, most preferably about 1143 nm.

The aspherical hollow silica have a short axis (e.g., a minimum outer particle width) of about 350 nm to about 1200 nm. It is understood that the short axis must be less than the long axis. Preferably, the short axis may be greater than about 570 nm, greater than about 700 nm, greater than about 750 nm, and less than about 810 nm, less than about 850 nm, and less than about 954 nm. More preferably, the short axis of the aspherical hollow silica particles may be about 720 nm to about 820 nm, most preferably about 770 nm.

The long axis may be about 1.3 times greater, about 1.4 times greater, about 1.6 times greater, or about 1.7 times greater than the short axis. Preferably, the ratio of long axis to short axis may be about 3:2 (e.g., about 1.5:1).

The aspherical hollow silica particles have a void long axis (e.g., a void length along the long axis of the aspherical hollow silica particle) of about 400 nm to about 1350 nm. Preferably, the void long axis may be greater than about 800 nm, greater than about 850 nm, greater than about 900 nm, and less than about 950 nm, less than about 1000 nm, and less than about 1100 nm. More preferably, the void long axis of the aspherical hollow silica particles may be about 842 nm to about 942 nm, most preferably about 892 nm.

The aspherical hollow silica particles have a void short axis (e.g., a void length along the short axis of the aspherical hollow silica particle) of about 200 nm to about 850 nm. It is understood that the void short axis must be less than the void long axis. Preferably, the void short axis may be greater than about 400 nm, greater than about 550 nm, greater than about 570 nm, and less than about 652 nm, less than about 750 nm, and less than about 800 nm. More preferably, the void short axis of the aspherical hollow silica particles may be about 540 nm to about 650 nm, most preferably about 595 nm.

The void long axis may be about 1.3 times greater, about 1.4 times greater, about 1.6 times greater, or about 1.7 times greater than the void short axis. Preferably, the ratio of void long axis to void short axis may be about 3:2 (e.g., about 1.5:1).

The aspherical hollow silica particles have a shell thickness of about 50 nm to about 300 nm. Preferably, the shell thickness may be greater than about 73 nm, greater than about 84 nm, greater than about 88 nm, and less than about 92 nm, less than about 100 nm, and less than about 200 nm. More preferably, the shell thickness of the aspherical hollow silica particles may be about 85 nm to about 95 nm, most preferably around 90 nm.

Aspherical hollow silica particles described herein may be used in sun care compositions. A sun care composition is a personal care composition for protecting a user from UV radiation. Examples of sun care compositions include compositions having an SPF rating (for example, sunscreen compositions) and/or personal care compositions where a UV blocker would be beneficial, such as, for example, moisturizers, lip balms, *etc*.

Presently described sun care compositions comprise aspherical hollow silica particles and at least one sunscreen active (one or more (*e.g.*, mixtures) sunscreen actives). Sunscreen actives is intended to include physical UV blockers (*e.g.*, titanium dioxide, zinc oxide) and chemical UV absorbers (*e.g.*, para-aminobenzoic acid, octyl methoxycinnamate). Examples of suitable sunscreen actives include titanium dioxide, zinc oxide, para-aminobenzoic acid, octyl methoxycinnamate, ethylhexyl methoxycinnamate, ethylhexyl salicylate, Octocrylene (2-ethylhexyl-2-cyano-3,3 diphenylacrylate), butyl methoxydibenzoylmethane, Avobenzone (4-t-butyl-4'-methoxydibenzoyl-methane), oxybenzone, dioxybenzone, cinoxate (2-ethoxyethyl-p-methoxy-cinnamate), diethanolamine-p-methoxycinnamate, ethylhexyl-p-methoxy-cinnamate, isopentenyl-4-methoxycinnamate, 2-ethylhexyl salicylate, digalloyl trioleate ethyl 4-bis(hydroxypropyl)aminobenzoate, glyceryl aminobenzoate, methyl anthranilate, homosalate (3,3,5-trimethylcyclohexyl salicylate), triethanolamine salicylate, 2-phenyl-benzimidazole-5-sulfonic acid, sulisobenzone (2-hydroxy-4-methoxy-benzophenone-5-sulfonic acid), Padimate A (amyl p-dimethylaminobenzoate), Padimate O (octyl dimethyl para aminobenzoate), 4-Methylbenzylidene camphor, sunscreen actives sold under the tradenames ECAMSULE™, TINOSORB™, NEO HELIOPAN™, MEXORYL™, BENZOPHENONE™, UVINUL™, UVASORB™, and/or PARSOL™, and/or mixtures thereof. Preferably, the sunscreen active is a mixture of avobenzone, octisalate, octocrylene, zinc oxide, titanium dioxide, and homosalate. More preferably, the sunscreen active is a mixture of avobenzone, octocrylene, homosalate, zinc oxide, titanium dioxide,and octisalate.

Preferably, the present sun care compositions contain greater than about 5 parts by weight (pbw) of the composition, greater than about 7 pbw, greater than or equal to about 10 pbw, and less than about 50 pbw, less than about 45 pbw, and less than or equal to about 40 pbw, total sunscreen active(s).

Preferably, the present sun care compositions contain greater than about 0.2 pbw of the composition, greater than about 0.5pbw, greater than or equal to about 1 pbw, and less than about 5 pbw, less than about 4.5 pbw, and less than or equal to about 4.0 pbw, aspherical hollow silica particles. More preferably, the present sun care compositions contain about 3 pbw aspherical hollow silica particles by weight of the composition.

Preferably, the present sun care compositions may comprise at least one of a cosmetically acceptable emollient, humectant, vitamin, moisturizer, conditioner, oil, silicone, suspending agent, carrier fluid, pigments, opacifier/pearlizer, surfactant, emulsifier, preservative, rheology modifier, colorant, pH adjustor, propellant, reducing agent, anti-oxidant, fragrance, foaming or de-foaming agent, tanning agent, insect repellant, and/or biocide. Preferably, the present sun care compositions may comprise at least one of a cosmetically acceptable emollient, humectant, vitamin, moisturizer, conditioner, oil, silicone, suspending agent, surfactant, emulsifier, preservative, rheology modifier, pH adjustor, reducing agent, anti-oxidant, and/or foaming or de-foaming agent. Preferably, a sun care composition may contain at least one of a humectant, a surfactant, and/or an emollient.

Aspherical hollow silica particles described herein may be used in sun care compositions as SPF boosters. Too high a concentration of sunscreen active results in impairment of the composition's aesthetics (such as tackiness, greasiness, grittiness, whiteness, etc.) and/or undesirable toxicological effects. Consequently, SPF boosters (e.g., compounds which are not recognized sunscreen actives, but work to increase the SPF) are added to sun care compositions to increase the SPF without adding more sunscreen actives. Preferably, the presently described aspherical hollow silica particles act as an SPF booster for sun care compositions. Preferably, the SPF boost ratio (per 3 wt.% booster) of the aspherical hollow silica particles in a sun care composition is more than about 2.5, more than about 3, more than about 3.5, and more preferably more than about 4.0, *e.g.*, as compared to a comparative composition without the aspherical hollow silica particles. SPF boost ratio may be determined using Equation 1 (Example 7).

In use, sun care compositions including the presently described aspherical hollow silica particles may be used to protect a mammal from damage caused by UV radiation (*e.g.*, UVA radiation and/or UVB radiation). For example, a method of protecting a mammal (*e.g.*, the skin of a mammal) from damage caused by UV radiation comprises applying sun care compositions including the presently described aspherical hollow silica particles to the skin of the mammal.

The following examples are for illustrative purposes only and are not intended to limit the scope of the appended claims.

## EXAMPLES

### Example 1

### Template Formation

Calcium carbonate ($CaCO_3$) templates were prepared as follows.

$CaCl_2$ solution was prepared by mixing 15.6 g $CaCl_2$ (anhydrous calcium chloride, Fisher Scientific) with 120 mL deionized water and 600 mL ethylene glycol (99%, Alfa Aesar) using magnetic stirring.

$NaHCO_3$ solution was prepared by mixing 24.4 g $NaHCO_3$ (Sodium bicarbonate, >99.5%, Sigma) with 240 mL deionized water and 1.2 L ethylene glycol using magnetic stirring. The $NaHCO_3$ solution was charged into a 4 L glass beaker and kept stirring using an overhead stirrer.

The CaCl$_2$ solution was slowly poured into the NaHCO$_3$ solution over a few minutes. The system turned cloudy during the addition. Stirring was stopped after 30 min (timed from the beginning of the addition of CaCl$_2$). The solution was evenly separated into four 1 L centrifuge bottles and centrifuged at 8000 rpm for 15 min. The supernatant was decanted. Approximately 300 g ethanol (200 proof, Pharmoca-Aaper) was added to each centrifuge bottle and mixed well with the solids before centrifuging again at 8000 rpm for 15 min. A second wash was performed by dispersing the product in 80 mL ethanol, separating them into four 45 mL centrifuge tubes and centrifugation at 12000 rpm for 15 min. The wet slurry was transferred to a glass container, heated at 80 °C under vacuum for 3 h to obtain dry powder comprising CaCO$_3$ templates characterized by an anisotropic shape with average long axis in the range of 500-1350 nm, and average short axis in the range of 200-850 nm.

## Example 2

### Aspherical Hollow Silica Particle Formation

Aspherical hollow silica particles were prepared as follows.

The CaCO$_3$ templates were synthesized substantially according to the process of Example 1. After the first wash, the CaCO$_3$ templates were dispersed in 76.5 mL ethanol and charged into a 250 mL round bottom flask forming a white suspension. The white suspension was kept stirring at 260 rpm using a stir plate, while 6.6 mL ammonium solution (ammonium hydroxide, 28-30 wt.%, Pharmco-Aaper) and 7.8 mL deionized water were added.

The system was allowed to mix for 10 min before feeding tetraethyl orthosilicate (TEOS) (Sigma) using a syringe pump at 13 mL/h for 30 min. The reaction was allowed to continue for another hour before quenched with 100 mL ethanol. After stirring, white solids slowly settled down to the bottom of the flask. The upper clear solution was decanted when the clear separation of solids and liquid was achieved. A triple wash was performed using 100 mL ethanol, 100 mL ethanol, and 100 mL deionized water.

Afterwards, 80 mL 1.5 M HCl solution (from 36.5-38 wt.% hydrochloric acid, Fisher Chemical) was slowly added to the solids while stirring at 260 rpm. Vigorous bubbling was observed upon the addition, then the white suspension turned translucent. The system was kept under stirring for 30 minutes before centrifugation at 10000 rpm for 10 min. The product was washed with 100 mL water twice and 100 mL ethanol once. The wet slurry was transferred to a glass container, heated at 110 °C under vacuum for 3 h to obtain dry powder.

## Example 3

### Aspherical Hollow Silica Particle Formation

Aspherical hollow silica particles were prepared substantially according to the process of Example 2 to afford Batches 1-5. All five batches were made using the template process of Example 1, size differences in Table 1 (below) may be attributable to particle size distribution.

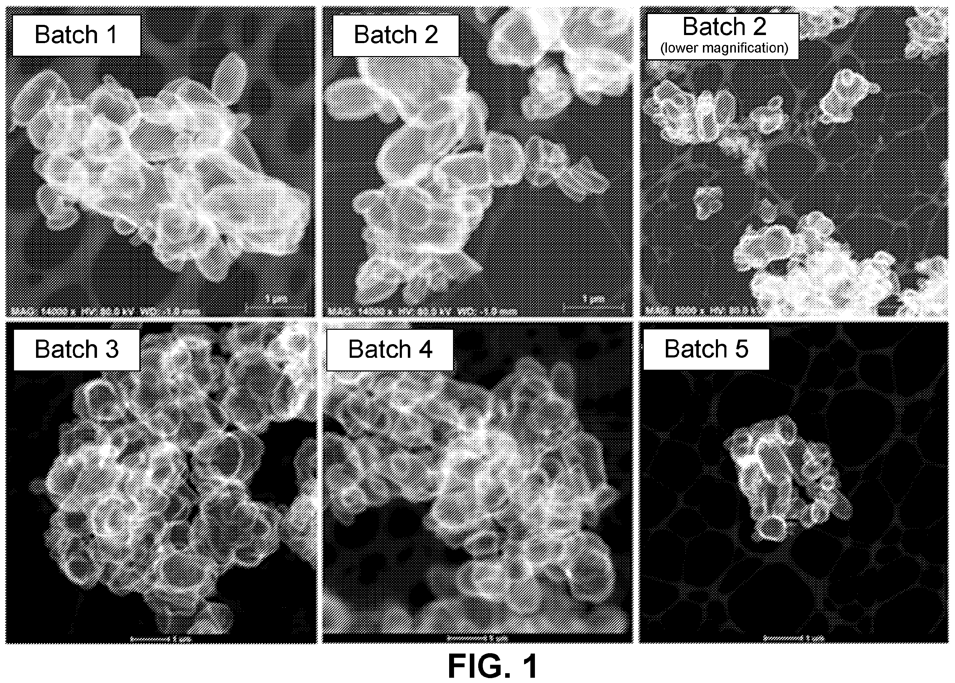

FIG. 1 is a group of Scanning Transmission Electron Microscope (STEM) images of aspherical hollow silica particles labeled Batches 1-5, demonstrating the hollow morphology of the spheres. Batch 1 and Batch 2 are shown at a first magnification. Batch 2 is then shown again at a relatively lower magnification. Batches 3-5 are shown at an intermediate magnification. STEM imaging was performed using a FEI Titan probe-corrected filed emission gun (FEG) transmission electron microscope (TEM) operated at an accelerating voltage of 200 keV. Images were collected at 2048 x 2048 image size, with a magnification range from 13kx - 34 kx.

Particle size measurements were performed manually with ImageJ software and results are summarized in Table 1. The aspherical hollow silica spheres in FIG. 1 have an anisotropic shape with an average long axis in the range of about 800 nm to about 1500 nm, and average short axis in the range of about 500 nm to about 1000 nm. The aspherical hollow silica spheres in FIG. 1 have relatively thin shells with a thickness varying between about 70 nm to about 120 nm.

Dimensions of aspherical hollow silica particles are shown in Table 1.

**TABLE 1**

|  | Particle long axis (nm) | Particle short axis (nm) | Void long axis (nm) | Void short axis (nm) | Shell thickness (nm) |
|---|---|---|---|---|---|
| Batch 1 | 1166.9±389.2 | 707.5±182.3 | 950.4±367.7 | 535.3±150.3 | 85.2±15.1 |
| Batch 2 | 1094.4±389.0 | 766.8±241.0 | 880.2±266.3 | 573.6±224.0 | 95.0±19.9 |
| Batch 3 | 1422.8±305.6 | 954.0±260.1 | 1102.1±334.9 | 793.7±247.1 | 85.0±13.6 |

(continued)

| | Particle long axis (nm) | Particle short axis (nm) | Void long axis (nm) | Void short axis (nm) | Shell thickness (nm) |
|---|---|---|---|---|---|
| Batch 4 | 1225.8±329.1 | 849.9±230.0 | 879.2±299.1 | 651.4±226.7 | 112.3±23.7 |
| Batch 5 | 807.7±230.3 | 570.1±126.8 | 651.8±229.6 | 419.7±113.3 | 73.8±13.8 |
| Average | 1143 | 770 | 892 | 595 | 90 |

In terms of morphology, the aspherical hollow silica particles are anisotropic and hollow. the aspherical hollow silica particles are relatively large, for example, with respect to average particle long axis, particle short axis, void long axis, and void short axis (1143nm, 770nm, 892nm, and 595nm, respectively).

**Example 4**

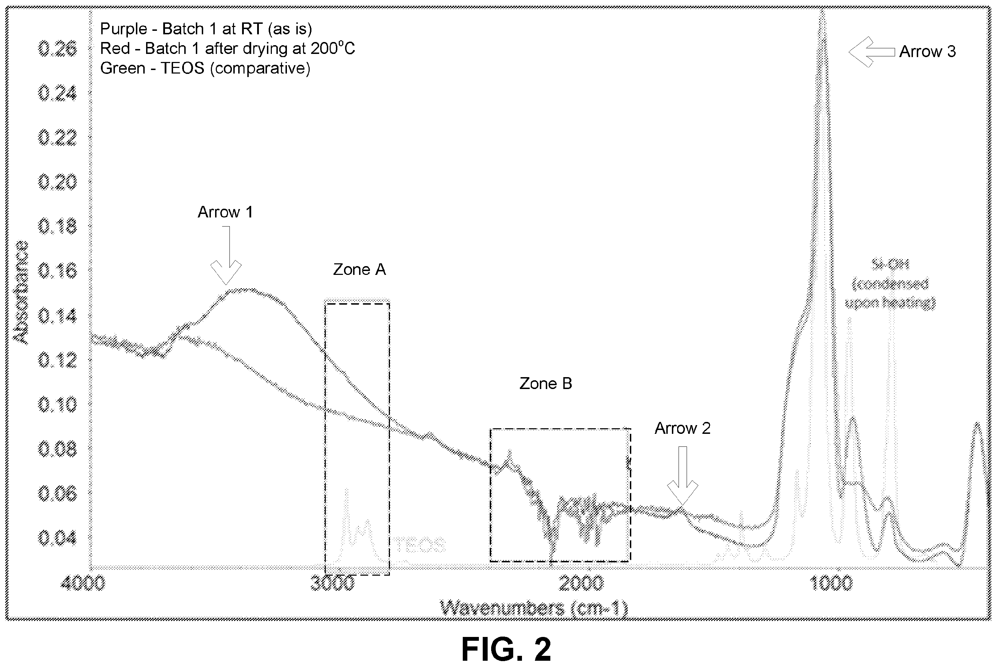

Aspherical hollow silica particles Batch 1 from Example 3 was characterized. FIG. 2 is an Attenuated Total Reflection Fourier Transform Infrared Spectroscopy (ATR-FTIR) spectra using a Thermo Nicolet iS-50 FTIR spectrometer with single bounce diamond ATR. Batch 1 aspherical hollow silica particles were analyzed at room temperature (RT) (purple line) and the same sample after drying at 200 °C (red line), along with a reference spectra of tetraethyl orthosilicate (TEOS) (green line).

Referring to Arrow 1, between wavenumbers 4000 and 3000, an O-H stretching mode appears in the room temperature Batch 1, due to the presence of water. Some portion of the peak may also be attributable to the presence of SiOH.

Referring to Zone A, C-H modes located near 3000 cm-1 as shown in the TEOS spectrum are not found in the aspherical hollow silica samples, thus indicating no detectable amount of C-H. Accordingly, since the detection limit of ATR-FTIR is usually 1 wt.%, the organic content in the aspherical hollow silica samples is below 1 wt.%, and conclusively may be stated as below 2 wt.% Compared to the RT treated sample, the 200 °C-treated sample showed significant reduction in the intensity of the broad peak above 3000 cm-1 due to the water removal.

Referring to Zone B, the lines display an artifact due to the ATR crystal.

Referring to Arrow 2, the small peak on the RT sample represents a water O-H bending mode.

Peaks observed below 2000 cm-1 are characteristic of various SI-O stretching modes. Arrow 3 is pointed at the strongest of these peaks.

Below 1000 cm-1, the RT sample and 200 °C-treated sample showed two Si-O stretching modes due to the condensation of hydroxyl groups. The TEOS sample showed relatively greater Si-OH stretching (condensed upon heating). Stated differently, the TEOS showed greatest peak intensity associated with the Si-OH stretching modes. The RT sample showed some degree of the modes, but the peaks are the least significant in the 200 °C dried sample because most of the Si-OH are condensed.

It should be noted that the sampling depth of ATR-FTIR is generally of the order of a few microns. Since the particle size of the aspherical hollow silica samples is smaller, e.g., on average, 1143 nm by 770 nm (see Table 1), ATR-FTIR measurement can be considered as a bulk measurement.

**Example 5 (Comparative)**

To ascertain the SPF of comparative sun care compositions, sunscreen formulations Comparative Batch A and Comparative Batch B were prepared having the ingredients as listed in Table 2.

**TABLE 2**

| Phase | Component | Comparative Batch A | Comparative Batch B |
|---|---|---|---|
| A | Deionized water | Balance | Balance |
| | ACULYN™ 38 Rheology Modifier (AcrylatesNinyl Neodecanoate Crosspolymer) thickener | 0.56 | 0.56 |
| | Butylene Glycol (1,3 Butanediol) emollient/humectant | 2.00 | 2.00 |
| | UCON™ 75-H-450 Fluid (PEG/PPG-17/6 Copolymer) emollient/sensory modifier | 0.5 | 0.5 |

(continued)

| Phase | Component | Comparative Batch A | Comparative Batch B |
|-------|-----------|---------------------|---------------------|
| | VERSENE™ disodium Ethylenediamine Tetraacetic Acetate (EDTA) chelant/mineral ion control | 0.10 | 0.10 |
| | SYMSAVE™ H Hydroxyacetophenone antioxidant/smoothing agent | 0.50 | 0.50 |
| | SUNSPHERES™ hollow polystyrene spheres SPF booster | - - | 3.00 |
| B | PROCOL™ CS20D Cetearyl Alcohol (and) Ceteareth 20 emulsifier | 1.75 | 1.75 |
| | ARLACEL™ 165 Glycerol Stearate (and) PEG-100 Stearate emulsifier | 2.00 | 2.00 |
| | RITAMOLLIENT™ CCT Caprylic/Capric Triglyceride emollient | 5.00 | 5.00 |
| | RITAMOLLIENT™ TN C12-15 Alkyl Benzoate emollient | 5.00 | 5.00 |
| | PARSOL™ HMS Homosalate sunscreen active | 5.00 | 5.00 |
| | PARSOL™ EHS Octisalate sunscreen active | 5.00 | 5.00 |
| | PARSOL™ 1789 Avobenzone sunscreen active | 3.00 | 3.00 |
| | PARSOL™ 340 Octocrylene sunscreen active | 4.00 | 4.00 |
| C | Triethanolamine 99% neutralizer | 0.4 | 0.4 |
| D | NEOLONE™ PH-100 Phenoxyethanol preservative | 0.5 | 0.5 |

Amounts are listed as parts by weight (pbw). Water is added so that the total equals 100 pbw (e.g., there is 3.0 pbw less water in Comparative Batch B).

Phase A components (except SYMSAVE™ H Hydroxyacetophenone antioxidant/smoothing agent) were mixed together and heated to 70 °C with agitation. At 70°C, the SYMSAVE™ H Hydroxyacetophenone antioxidant/smoothing agent was added to the Phase A vessel and the contents mixed until fully dissolved.

In a separate vessel, Phase B components were mixed together and heated to 75 °C until all ingredients were melted or dissolved. With agitation (e.g., 500 rpm if no splash), Phase B was gradually mixed into Phase A at 70 °C over a 5 min period. With homogenization, of half of Phase C was added to the A/B mixture. The mixture was then homogenized at high speed for 3 mins, before switching to an overhead stirrer at 400 rpm. The other half of Phase C was subsequently mixed into the formulation.

Heat and stirring were turned off of the A/B/C mixture, and it was allowed to cool down to 45°C. Phase D was added and the high shear mixing was continued until the formulation reached room temperature.

Comparative Batch A has no SPF boosters. Comparative Batch B has SUNSPHERES™ hollow polystyrene spheres, an SPF booster.

**Example 6**

To ascertain the SPF of sun care compositions, sunscreen formulations Batch C and Batch D were prepared having the ingredients as listed in Table 3.

**TABLE 3**

| Phase | Component | Batch C | Batch D |
|---|---|---|---|
| A | Deionized water | Balance | Balance |
| | ACULYN™ 38 Rheology Modifier (Acrylates/Vinyl Neodecanoate Crosspolymer) thickener | 0.56 | 0.56 |
| | Butylene Glycol (1,3 Butanediol) emollient/humectant | 2.00 | 2.00 |
| | UCON™ 75-H-450 Fluid (PEG/PPG-17/6 Copolymer) emollient/sensory modifier | 0.5 | 0.5 |
| | VERSENE™ disodium Ethylenediamine Tetraacetic Acetate (EDTA) chelant/mineral ion control | 0.10 | 0.10 |
| | SYMSAVE™ H Hydroxyacetophenone antioxidant/smoothing agent | 0.50 | 0.50 |
| | $CaCO_3$ Template (prepared substantially as described in Example 1) | 3.00 | - - |
| | Aspherical hollow silica particles (combined Batches 1, 2, 3, 4, and 5 from Example 3) | - | 3.00 |
| B | PROCOL™ CS20D Cetearyl Alcohol (and) Ceteareth 20 emulsifier | 1.75 | 1.75 |
| | ARLACEL™ 165 Glycerol Stearate (and) PEG-100 Stearate emulsifier | 2.00 | 2.00 |
| | RITAMOLLIENT™ CCT Caprylic/Capric Triglyceride emollient | 5.00 | 5.00 |
| | RITAMOLLIENT™ TN C12-15 Alkyl Benzoate emollient | 5.00 | 5.00 |
| | PARSOL™ HMS Homosalate sunscreen active | 5.00 | 5.00 |
| | PARSOL™ EHS Octisalate sunscreen active | 5.00 | 5.00 |
| | PARSOL™ 1789 Avobenzone sunscreen active | 3.00 | 3.00 |
| | PARSOL™ 340 Octocrylene sunscreen active | 4.00 | 4.00 |
| C | Triethanolamine 99% neutralizer | 0.4 | 0.4 |
| D | NEOLONE™ PH-100 Phenoxyethanol preservative | 0.5 | 0.5 |

Amounts are listed as parts by weight (pbw). Water is added so that the total equals 100 pbw.

Phase A components (except SYMSAVE™ H Hydroxyacetophenone antioxidant/smoothing agent) were mixed together and heated to 70 °C with agitation. At 70 °C, the SYMSAVE™ H Hydroxyacetophenone antioxidant/smoothing agent was to added to the Phase A vessel and the contents mixed until fully dissolved.

In a separate vessel, Phase B components were mixed together and heated to 75 °C until all ingredients were melted or dissolved. With agitation (e.g., 500 rpm if no splash), Phase B was gradually mixed into Phase A at 70 °C over a 5 min period. With homogenization, of half of Phase C was added to the A/B mixture. The mixture was then homogenized at high speed for 3 mins, before switching to an overhead stirrer at 400 rpm. The other half of Phase C was subsequently mixed into the formulation.

Heat and stirring were turned off of the A/B/C mixture, and it was allowed to cool down to 45 °C. Phase D was added and the high shear mixing was continued until the formulation reached room temperature.

Batch C contains the $CaCO_3$ template material. Batch D contains a mixture of aspherical hollow silica particles Batches 1-5 (Table 1), in order to provide enough quantity of material for the test.

**Example 7**

32.5 mg of the respective sun care compositions from Examples 5 and 6 were each coated on a 5cm x 5cm PMMA plate using a wire round rod. The drawdown film was allowed to dry for at least 30 mins before SPF measurements are taken to allow adequate water evaporation.

*In vitro* SPF was determined using a UV-2000S SPF Analyzer with an integrating sphere and SPF Operating Software supplied by Labsphere (North Sutton, NH, USA). The UV-2000S measured the UV absorbance spectrum of the drawdown sunscreen film over UV radiation wavelengths (290-400 nm) and calculated an SPF value based on the UV absorbance spectrum. Nine data points were collected (e.g., from different locations on the film designated by the UV-2000S), with three repeats for each formulation.

The sunscreen formulations were heat aged at 45 °C for 2 weeks, 1 month, and 2 months to test the SPF.

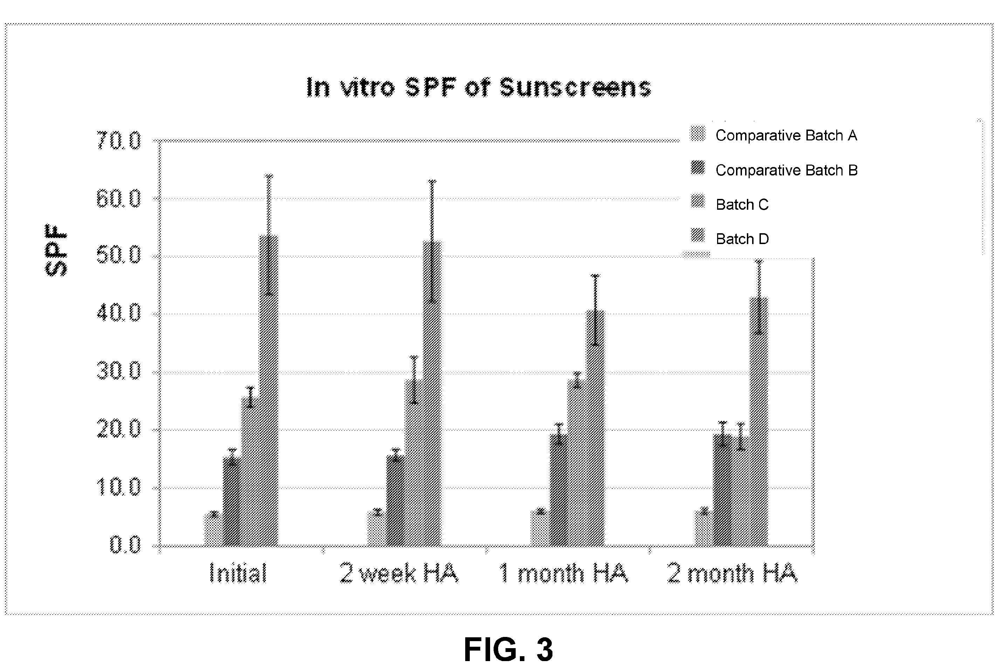

FIG. 3 is a diagram of SPF measurements for initial and heat aged formulations including comparative sun care formulations (e.g., Comparative Batch A (no SPF booster) and Comparative Batch B (a conventional SPF booster), a sun care formulation incorporating template material (Batch C), and a sun care formulation that includes aspherical hollow silica particles (e.g., as an SPF booster) (Batch D). FIG. 3 shows SPF for Comparative Batch A, Comparative Batch B, Batch C, and Batch D, respectively, at each time period. Results are also shown in Table 4.

TABLE 4

|  | Initial SPF | SPF after 2 weeks heat age at 45°C | SPF after 1 month heat age at 45°C | SPF after 2 month heat age at 45°C |
|---|---|---|---|---|
| Comparative Batch A | 5.52 | 5.84 | 6.00 | 6.04 |
| Comparative Batch B | 15.37 | 15.71 | 19.30 | 19.30 |
| Batch C | 25.72 | 28.67 | 28.67 | 18.89 |
| Batch D | 53.73 | 52.64 | 40.73 | 43.00 |

$CaCO_3$ particles provided good SPF boost. Aspherical hollow silica particles (Batch D) surprisingly delivered very high SPF boost efficiency, four times higher than the commercial benchmark (Comparative Batch B) before heat aging. After 2 month heat aging, the SPF boost ratio of Batch D remained three times higher than the commercial benchmark (Comparative Batch B).

To compare compositions, an SPF boost ratio was calculated using **Equation 1.**

$$SPF\ boost\ ratio = \frac{SPF(sample) - SPF\ (no\ booster)}{SPF\ (no\ booster)} \qquad Eq.\ 1$$

The results of the SPF boost ratio calculation are shown in TABLE 5.

TABLE 5

| Sun Care Composition | SPF boost ratio (per 3 wt.% hollow silica) | | |
|---|---|---|---|
|  | Initial | 1 month heat age at 45°C | 2 month heat age at 45°C |
| Comparative Batch B | 1.8 | 2.2 | 2.2 |
| Batch D | 8.7 | 5.8 | 6.1 |

Accordingly, Batch D is a better SPF booster than Comparative Batch B, both initially, after one month, and after two months of heat aging at 45°C.

It is understood that this disclosure is not limited to the embodiments specifically disclosed and exemplified herein. Various modifications of the invention will be apparent to those skilled in the art. Such changes and modifications may be made without departing from the scope of the appended claims. Moreover, each recited range includes all combinations and sub-combinations of ranges, as well as specific numerals contained therein.

**Claims**

1. A sun care composition, comprising:

   aspherical hollow silica particles having a long axis and a short axis, wherein the average short axis is greater than 400 nm; and
   at least one sunscreen active.

2. The sun care composition of claim 1, wherein the aspherical hollow silica particles have a shell thickness of greater than about 50 nm.

3. The sun care composition according to any of the preceding claims, wherein the aspherical hollow silica particles have a long axis that is at least 1.25 times greater than the short axis.

4. The sun care composition according to any of the preceding claims, wherein the aspherical hollow silica particles have an average long axis greater than 500 nm.

5. The sun care composition according to any of the preceding claims, wherein the aspherical hollow silica particles have an average void long axis greater than 350 nm.

6. The sun care composition according to any of the preceding claims, wherein the aspherical hollow silica particles have an average void short axis less than 1100 nm.

7. The sun care composition according to any of the preceding claims, wherein a sun protection factor (SPF) boost of the sun care composition is more than about 3.

8. The sun care composition according to any of the preceding claims, further comprising hollow polystyrene spheres.

9. The sun care composition according to any of the preceding claims, further comprising at least one of a cosmetically acceptable emollient, carrier fluid, pigment, humectant, vitamin, moisturizer, conditioner, oil, silicone, suspending agent, surfactant, emulsifier, preservative, rheology modifier, pH adjustor, reducing agent, anti-oxidant, and/or foaming or de-foaming agent.

10. Use of aspherical hollow silica particles having a long axis and a short axis, wherein the average short axis is greater than 400 nm, in a sun care composition as an SPF booster.

**Patentansprüche**

1. Sonnenpflegezusammensetzung, umfassend:

   asphärische hohle Siliziumdioxidteilchen mit einer langen und einer kurzen Achse, wobei die durchschnittliche kurze Achse größer als 400 nm ist, und
   mindestens einen Sonnenschutzwirkstoff.

2. Sonnenpflegezusammensetzung nach Anspruch 1, wobei die asphärischen hohlen Siliziumdioxidteilchen eine Schalendicke von mehr als etwa 50 nm aufweisen.

3. Sonnenpflegezusammensetzung nach einem der vorstehenden Ansprüche, wobei die asphärischen hohlen Siliziumdioxidteilchen eine lange Achse aufweisen, die mindestens 1,25 Mal größer ist als die kurze Achse.

4. Sonnenpflegezusammensetzung nach einem der vorstehenden Ansprüche, wobei die asphärischen hohlen Siliziumdioxidteilchen eine durchschnittliche lange Achse von mehr als 500 nm aufweisen.

5. Sonnenpflegezusammensetzung nach einem der vorstehenden Ansprüche, wobei die asphärischen hohlen Siliziumdioxidteilchen eine durchschnittliche Hohlraumlängenachse von mehr als 350 nm aufweisen.

6. Sonnenpflegezusammensetzung nach einem der vorstehenden Ansprüche, wobei die asphärischen hohlen Siliziumdioxidteilchen eine durchschnittliche Hohlraumkurzachse von weniger als 1100 nm aufweisen.

7. Sonnenpflegezusammensetzung nach einem der vorstehenden Ansprüche, wobei eine Verstärkung des Lichtschutzfaktors (LSF) der Sonnenpflegezusammensetzung mehr als etwa 3 beträgt.

8. Sonnenpflegezusammensetzung nach einem der vorstehenden Ansprüche, ferner umfassend Polystyrolhohlkugeln.

9. Sonnenpflegezusammensetzung nach einem der vorstehenden Ansprüche, ferner umfassend mindestens einen kosmetisch verträglichen Weichmacher, Trägerflüssigkeit, Pigment, Feuchthaltemittel, Vitamin, Feuchtigkeitsspender, Conditioner, Öl, Silikon, Suspensionsmittel, Tensid, Emulgator, Konservierungsmittel, Rheologiemodifikator, pH-Regler, Reduktionsmittel, Antioxidationsmittel und/oder Schaumbildner oder Entschäumer.

10. Verwendung asphärischer hohler Siliziumdioxidteilchen mit einer langen und einer kurzen Achse, wobei die durchschnittliche kurze Achse größer als 400 nm ist, in einer Sonnenpflegezusammensetzung als LSF-Verstärkung.

**Revendications**

1. Composition de protection solaire, comprenant :

   des particules de silice creuses asphériques ayant un grand axe et un petit axe, dans laquelle le petit axe moyen est supérieur à 400 nm ; et
   au moins un actif d'écran solaire.

2. Composition de protection solaire selon la revendication 1, dans laquelle les particules de silice creuses asphériques ont une épaisseur d'enveloppe supérieure à environ 50 nm.

3. Composition de protection solaire selon l'une quelconque des revendications précédentes, dans laquelle les particules de silice creuses asphériques ont un grand axe qui est au moins 1,25 fois plus grand que le petit axe.

4. Composition de protection solaire selon l'une quelconque des revendications précédentes, dans laquelle les particules de silice creuses asphériques ont un grand axe moyen supérieur à 500 nm.

5. Composition de protection solaire selon l'une quelconque des revendications précédentes, dans laquelle les particules de silice creuses asphériques ont un grand axe de vide moyen supérieur à 350 nm.

6. Composition de protection solaire selon l'une quelconque des revendications précédentes, dans laquelle les particules de silice creuses asphériques ont un petit axe moyen inférieur à 1 100 nm.

7. Composition de protection solaire selon l'une quelconque des revendications précédentes, dans laquelle une amélioration du facteur de protection solaire (FPS) de la composition solaire est supérieure à environ 3.

8. Composition de protection solaire selon l'une quelconque des revendications précédentes, comprenant en outre des sphères creuses de polystyrène.

9. Composition de protection solaire selon l'une quelconque des revendications précédentes, comprenant en outre au moins l'un parmi un émollient, un fluide porteur, un pigment, un humectant, une vitamine, un hydratant, un revitalisant, une huile, une silicone, un agent de suspension, un agent tensioactif, un émulsifiant, un conservateur, un modificateur de rhéologie, un ajusteur de pH, un agent réducteur, un antioxydant, et/ou un agent moussant ou antimousse, cosmétiquement acceptable.

10. Utilisation de particules de silice creuses asphériques ayant un grand axe et un petit axe, dans laquelle le petit axe moyen est supérieur à 400 nm, dans une composition de protection solaire comme agent d'amélioration du FPS.

FIG. 1

EP 4 236 906 B1

**FIG. 2**

EP 4 236 906 B1

FIG. 3

EP 4 236 906 B1

## REFERENCES CITED IN THE DESCRIPTION

**Patent documents cited in the description**

- US 63106645
- US 2020237632 A1

- WO 2014203913 A1

**Non-patent literature cited in the description**

- **MASAYOSHI FUJI et al.** Development of new templating approach for hollow nanoparticles and their applications. *ADVANCED POWDER TECHNOLOGY*, 01 January 2014, vol. 25 (1), 91-100